(19) Europäisches Patentamt
European Patent Office
Office européen des brevets

(11) Veröffentlichungsnummer: **0 505 596 B1**

(12) **EUROPÄISCHE PATENTSCHRIFT**

(45) Veröffentlichungstag der Patentschrift: **26.04.95**

(51) Int. Cl.⁶: **A61K 7/48**

(21) Anmeldenummer: **91104968.2**

(22) Anmeldetag: **28.03.91**

(54) **Verfahren zur Herstellung eines Feuchthaltemittels.**

(43) Veröffentlichungstag der Anmeldung:
**30.09.92 Patentblatt 92/40**

(45) Bekanntmachung des Hinweises auf die
Patenterteilung:
**26.04.95 Patentblatt 95/17**

(84) Benannte Vertragsstaaten:
**BE DE FR GB IT NL**

(56) Entgegenhaltungen:

BECKER, D ET AL. 'technologie des zuk-
kers'&& H. SCHAPER

CHEMICAL ABSTRACTS, vol. 83, no. 14, 1974,
Columbus, Ohio, US;abstract no. 117539N,

Chemiker Kalender, Hg. H.U.Vogel, 2.Aufla-
ge,Springer Verlag (1974), Seite 286/287

Römpps Chemie Lexikon, O.A.Neumüller,
Franckh'sche Verlagsbuchhandlung, 9.Aufla-
ge (1990), Seite 2927

(73) Patentinhaber: **AMINO GmbH**
**Postfach 20**
**D-38373 Frellstedt (DE)**

(72) Erfinder: **Steinmetzer, Walter**
**Im Bodetal 10**
**W-3334 Süpplingen (DE)**

(74) Vertreter: **Einsel, Martin et al**
**Patentanwalt**
**Dipl.-Phys. M. Einsel**
**Jasperallee 1a**
**D-38102 Braunschweig (DE)**

**Beschreibung**

Die Erfindung betrifft ein Verfahren zur Herstellung eines Feuchthaltemittels.

Feuchthaltemittel oder Feuchtigkeitsstabilisatoren werden bei der Herstellung von Lebensmitteln und industriellen Erzeugnissen eingesetzt, um ein Austrocknen der Produkte zu verhindern. Ein Hauptanwendungsgebiet finden diese Feuchtigkeitsstabilisatoren außerdem in Hautpflegemitteln, um die Haut feucht und geschmeidig zu halten. In der Oberfläche der menschlichen Haut, der Epidermis, sind natürliche Feuchtigkeitsregulatoren vorhanden. Diese Feuchtigkeitsregulatoren - mit der Bezeichnung Natural Moisturizing Factor NMF - sind in der einschlägigen Literatur, etwa Römpp Chemie Lexikon 9. Auflage (1990) Band 2, Seite 1347, ausführlich beschrieben und bestehen zu 40 % aus freien Aminosäuren, zu 12 % aus L-2-Pyrrolidon-5-Carbonsäure, 12 % Lactat, 7 % Harnstoff, 1,5 % Harnsäure, Glukosamin, Keratin, Ammonium, Natrium, Kalium, Magnesium und Kalziumzitraten, Formiaten, Phosphaten und Chloriden.

Neben der feuchtigkeitsregulierenden Wirkung haben die organischen Säuren noch eine Schutzfunktion für die Haut, da sie einen natürlichen Säuremantel bilden. Diese natürlichen Feuchthaltestoffe werden durch Seifen oder andere Tenside herausgelöst, und die Folge ist ein Austrocknen und Schuppen der Haut. Durch synthetische Feuchthaltemittel, wie Glykole, Glyzerin oder Hydrolysate von Keratin oder kollagenhaltigen Stoffen, sollen in Hautpflegemitteln diese natürlichen Feuchtigkeitsregulatoren ersetzt werden.

Wünschenswert wäre es jedoch, zumindest als Alternative für die synthetischen Feuchthaltemittel auch natürliche Feuchthaltemittel bzw. gleiche oder eine ähnliche chemische Zusammensetzung wie die natürlichen Feuchthaltemittel aufweisende Möglichkeiten zu gewinnen.

In einem Aufsatz von Klaus Hoffmann, Lactil - ein neuer Feuchthaltekomplex, in: Seifen-Öle-Fette-Wachse 103 (1977), 7, wird die Bedeutung von 2-Pyrrolidon-5-Carbonsäure als natürliche, hauteigene Substanz herausgestellt und für den Einsatz in hydroregulativen Kosmetika vorgeschlagen. Problematisch bleibt die Gewinnung von geeigneten Feuchthaltemitteln aus natürlichen Produkten.

Aufgabe der Erfindung ist es daher, ein Verfahren zur Herstellung eines Feuchthaltemittels vorzuschlagen, mit dem den natürlichen Feuchtigkeitsregulatoren chemisch ähnliche Möglichkeiten zur Verfügung gestellt werden können.

Diese Aufgabe wird dadurch gelöst, daß zuckerfreie oder teilentzuckerte Restmelasse aus Zuckerrübenmelasse einer Behandlung unterworfen wird, bei der enthaltene Kaliumsalze von organischen Säuren, insbesondere von L-2-Pyrrolidon-5-Carbonsäure und von Milchsäure in diese freien Säuren überführt, die Nebenprodukte von den freien Säuren getrennt, diese neutralisiert, entfärbt und zu einem Feuchthaltemittel konzentiert werden.

Dieses neuartige Verfahren ermöglicht es, aus den Nichtzuckerstoffen der Zuckerrübenmelasse, also gewissermaßen einem Abfallprodukt bei der Zuckerherstellung, natürliche Feuchthalteregulatoren zu gewinnen.

Die Erfindung stützt sich dabei auf die Entdeckung, daß in der Zuckerrübenmelasse die natürlichen Feuchtigkeitsstabilisatoren L-2-Pyrrolidon-5-Carbonsäure, Milchsäure, Citronensäure und andere organische Säuren sowie freie Aminosäuren enthalten sind, und zwar in Form von Kaliumsalzen und in einem Verhältnis, das den gewünschten natürlichen Feuchtigkeitsregulatoren nahekommt.

Durch die erfindungsgemäßen Verfahrensschritte ist es möglich, diese natürlichen Feuchtigkeitsregulatoren aus der Zuckerrübenmelasse zu isolieren.

Die Zuckerrübenmelasse ist ein Sirup mit so geringer Reinheit, daß beim weiteren Konzentrieren kein Zucker mehr auskristalliert. Die Zuckerrübenmelasse hat einen Trockenstoffgehalt von ca. 78 % und enthält neben 48 % Saccharose noch 30 % organische und anorganische Nichtzuckerstoffe. Dies sind im wesentlichen die Kalium- und Natriumsalze von verschiedenen organischen Säuren sowie freie Aminosäuren, Betain und andere organische Substanzen.

Die Zuckerrübenmelasse dient als Rohstoff für verschiedene biotechnologische Verfahren, wie Ethanol, Citronensäure, Glukonsäure, Glutaminsäure und Backhefegewinnung. Für die Isolierung der Saccharose aus der Zuckerrübenmelasse stehen verschiedene chemische und chromatographische Verfahren zur Verfügung. Bei all diesen Prozessen verbleiben Restmelassen, welche die Nichtzuckerstoffe in angereicherter Form enthalten. Diese Restmelassen, auch Vinassen oder Melasseschlempe genannt, sind die Rohstoffe für die vorliegende Erfindung. Bevorzugter Ausgangsstoff sind jedoch die bei der chromatographischen Trennung der Zuckerrübenmelasse anfallenden Restmelassen. Bei der chromatographischen Trennung nach dem Ionenausschlußverfahren werden die polaren Nichtzuckerstoffe in der ersten Fraktion, gefolgt von einer Saccharose- Fraktion und einer Betain-Fraktion erhalten. In der ersten Nichtzuckerstoff-Fraktion sind die Kalisalze der Pyrrolidoncarbonsäure, Milchsäure und andere organische Säuren neben einigen Aminosäuren, wie Serin und Threonin, angereichert.Diese beiden Aminosäuren sind gleichfalls für die Erhaltung der Hautfunktion als Feuchtigkeitsregulator von Bedeutung. Weitere Bestandteile dieser teilentzuckerten Melasse sind die Melassefarbstoffe, wie Melanine, Melanoidine und Karamelfarbstoffe und restliche Anteile von Saccharose, Raffinose und Monosaccharide.

Drei alternative Möglichkeiten zur Durchführung des Verfahrens haben sich als besonders aussichtsreich erwiesen.

Bei der ersten Alternative wird so vorgegangen, daß die Restmelasse auf einen pH-Wert von mindestens 3,0, insbesondere von 1,5 bis 2,0 angesäuert und mit einem organischen Lösungsmittel die organischen Säuren, insbesondere L-2-Pyrrolidon-5-Carbonsäure und Milchsäure, extrahiert, neutralisiert und zu einem Feuchthaltemittel konzentriert werden.

Dieses Verfahren hat sich als insbesondere wenig aufwendig herausgestellt. Zum Ansäuern ist der Einsatz von Schwefelsäure bevorzugt. Es bildet sich $K_2SO_4$. Mit einem organischen Lösungsmittel, besonders bevorzugt mit Butanol oder Isobutanol, werden dann die entstehenden organischen Säuren, insbesondere $H^+$- $PCS^-$, gewonnen. Die als Kaliumsalz vorliegenden organischen Säuren können unmittelbar nicht aus den Restmelassen abgetrennt werden, als freie organische Säuren dagegen insbesondere mit Butanol und Isobutanol durchaus.

Bei einer zweiten Alternative wird so vorgegangen, daß die Restmelasse über einen Kationenaustauscher geführt und die Kaliumionen an diesen gebunden und anschließend die entstehenden freien organischen Säuren extrahiert werden.

Dabei ist insbesondere ein Kationenaustauscher in H-Form bevorzugt. Die Kaliumionen der Kaliumsalze werden an den Austauscher gebunden, wodurch die organischen Säuren in ihrer freien Form entstehen und wiederum extrahiert werden können, insbesondere mit Butanol oder Isobutanol.

Mit diesem Verfahren sind noch bessere Ausbeutegrade und Reinheiten erzielt worden, da neben den Kaliumionen auch störende Farbstoffe vom Austauscher adsorbiert werden.

Eine dritte Alternative besteht darin, daß die Restmelasse über einen Kationenaustauscher geführt und die Kaliumionen an diesen gebunden und anschließend das verbleibende Gemisch über einen Anionenaustauscher geführt und die entstandenen freien organischen Säuren an diesen gebunden werden, daß das jetzt verbleibende Gemisch entfernt wird und anschließend die organischen Säuren, insbesondere L-2-Pyrrolidon-5-Carbonsäure und Milchsäure, vom Anionenaustauscher eluiert, neutralisiert und zu einem Feuchthaltemittel konzentiert werden.

Dieses Verfahren hat sich als die beste Methode vorläufig herausgestellt. Während zunächst, wie bei der zweiten Alternative ein Kationenaustauscher bevorzugt in H-Form eingesetzt wird, werden aus dem entstehenden Gemisch nun jedoch nicht unmittelbar die organischen Säuren extrahiert. Stattdessen wird das verbleibende Gemisch über einen Anionenaustauscher geführt, wobei die soeben entstandenen freien organischen Säuren nun an diesen gebunden werden. Sämtliche nun noch im Gemisch verbliebenen Substanzen sind Nebenprodukte und können ablaufen. Die organischen Säuren, insbesondere die L-2-Pyrrolidon-5-Carbonsäure und die Milchesäure, können nun wiederum vom Anionenaustauscher abgetrennt werden, bevorzugt durch Schwefelsäure. Sie liegen damit unmittelbar vor und können neutralisiert und zu einem Feuchthaltemittel konzentriert werden.

Unter Umständen ist es bei diesem Verfahren ebenfalls noch erforderlich, aus diesem Endprodukt eine Extraktion vorzunehmen, die wiederum bevorzugt mit Butanol oder Isobutanol durchgeführt würde. Dies hängt etwas von der Art der Restmelasse ab, mit der das Verfahren gestartet wird. Insbesondere bei verhältnismäßig rein vorliegenden Phasen der Restmelasse haben Versuche jedoch ergeben, daß eine Extraktion nicht erforderlich ist, wenn diese Verfahrensvariante gewählt wird.

Besonders gut läßt sich das Verfahren mit einer Extraktion der freien organischen Säuren mittels Butanol oder Isobutanol durchführen. Beim Ansäuern einer Restmelasse auf einen pH-Wert von insbesondere 1,5 bis 2,0 werden die L-Pyrrolidoncarbonsäure, die Milchsäure und andere organische Säuren vom Zucker und dem Kalisalz abgetrennt.

Bei einer solchen erfindungsgemäßen Extraktion der organischen Säuren gelangt unter Umständen gleichfalls ein Teil der Melassefarbstoffe in die organische Phase. Die Lösungen der organischen Säuren sind daher nach Abdestillation des Lösungsmittels dunkel gefärbt, und in der wässrigen Phase sind die restlichen Farbstoffe neben Kalisalz und Zucker enthalten.

Zur weiteren Verbesserung des erfindungsgemäßen Verfahrens ist daher eine dreistufige Ausführungsform entwickelt worden, welche im folgenden beschrieben wird. Aus dieser Beschreibung gehen dabei zugleich bevorzugte Möglichkeiten des Verfahrens hervor, da die verschiedenen Schritte, zumindest teilweise, auch unabhängig voneinander durchgeführt werden können. So hat sich zum Ansäuern der Restmelassen insbesondere Schwefelsäure als geeignet erwiesen.

In der ersten Trennstufe wird die Restmelasse mit einem stark sauren, bevorzugt makroporösen Kationenaustauscher in H-Form behandelt. Die Restmelasse mit ca. 10 % Trockensubstanz wird dabei mit einer Geschwindigkeit von 5 - 10 Bettvolumen pro Stunde über den Kationenaustauscher geleitet, bis im Auslauf ein pH-Wert von 5- 6 erreicht wird. Dabei sind alle Wasserstoffionen vollständig durch Kaliumionen ausgetauscht, und der Austauscher wird dann mit 4 %iger Natronlauge eluiert und die Farbstoffe abgelöst. Nach dem Nachwaschen mit Wasser wird der Austauscher erneut mit Schwefelsäure regeneriert. Im Auslauf des Kationenaustauschers werden folgende Fraktionen aufgefangen:

Fraktion 1 bis pH 2,5 für die Gewinnung der organischen Säuren,

Fraktion 2 von pH 2,5 bis 6,0 Rückführung zum nächsten Austauscherzyklus,

Fraktion 3 von pH 6,0 bis 9,0 Farbstoff-Fraktion

Fraktion 4 Kaliumsulfatlösung für die Kaliumsulfatherstellung,

Fraktion 5 Schwefelsäure zur Vorregeneration für den nächsten Zyklus.

Wahlweise kann nunmehr die erste Fraktion nach dem Konzentrieren mit Butanol oder Isobutanol extrahiert werden. Dabei verbleibt der Zucker mit einem Teil der Farbstoffe und nicht extrahierbaren Substanzen der wässrigen Phase, und die organischen Säuren in der Butanolphase werden nach Neutralisation mit Wasser extrahiert und Isobutanol in den Prozeß zurückgeführt. Die wässrige Phase der Salze von organischen Säuren wird konzentiert und ein Feuchthaltemittel gewonnen.

Vorzugsweise wird jedoch die erste Fraktion aus dem Kationenaustauscher in der nächsten Trennstufe über einen schwachbasischen bevorzugt makroporösen Anionenaustauscher behandelt. Die organischen Säuren werden dabei gemeinsam mit den Farbstoffen am Austauscher gebunden, und im Auslauf des Anionenaustauschers wird eine entfärbte salzfreie Zuckerlösung erhalten. Bei Erreichung eines pH-Wertes von 6,0 im Auslauf wird der Anionenaustauscher abgesüßt und die organischen Säuren mit verdünnter Schwefelsäure oder Salzsäure eluiert, mit Wasser nachgewaschen, und anschließend wird dann der Anionenaustauscher mit Ammoniak regeneriert.

Durch die erfindungsgemäße Eluierung der organischen Säuren vom Anionenaustauscher mit verdünnten anorganischen Säuren werden die organischen Säuren in freier Form erhalten und die aktiven Gruppen des Anionenaustauschers mit Sulfat und Chloridionen abgesättigt, ohne daß die adsorbierten Farbstoffe abgelöst werden. Dieser überraschende und nicht vorhersehbare Effekt führt dazu, daß man die organischen Säuren in einer hellgefärbten Lösung erhält und eine nachfolgende Extraktion mit einem organischen Lösungsmittel bei einigen Restmelassen überflüssig wird.

Neben der Gewinnung von reinen Zuckerlösungen und reinen Lösungen der organischen Säuren wird in dieser Trennstufe eine konzentrierte Farbstofflösung bei der Regeneration mit Ammoniak erhalten. Beim Eluieren der organischen Säuren vom Anionenaustauscher mittels verdünnter Schwefelsäure wird die überschüssige Schwefelsäure durch Zusatz von Kalziumcarbonat oder Kalziumhydroxid neutralisiert und nach dem Eindampfen als Kalziumsulfat abfiltert. In einer weiteren Reinigungsstufe können auch diese Lösungen einer Butanolextraktion unterworfen werden. Wie in den nun folgenden Ausführungsbeispielen erläutert wird, kann jedoch nach jeder Trennstufe die Extraktion der organischen Säuren bei einem pH-Wert von mindestens 3,0, insbesondere von 1,5 bis 2,0, mit dem organischen Lösungsmittel vorgenommen werden.

Als konkrete Möglichkeiten zur Durchführung des erfindungsgemäßen Verfahrens seien im folgenden 3 Ausführungsbeispiele mit den erhaltenen Feuchthaltemitteln aufgeführt:

Ausführungsbeispiel 1:

1000 g Dickschlempe mit 70 % Trockensubstanz aus der Etanol-Herstellung werden mit 38 g 5 molarer Schwefelsäure auf einen pH-Wert von 1,7 eingestellt und 60Min. bei 120°C im Autoklav zwecks Zerstörung von Farbstoffen erhitzt, und nach dem Abkühlen werden ca. 300 g eines Gemisches von Kaliumsulfat und Huminstoffen abfiltert. Die saure filtrierte Lösung wird dann zweimal mit je 500 ml Isobutanol extrahiert, die Butanolphase mit 1 molarer Natronlauge auf einen pH-Wert von 6 bis 7 eingestellt, die wässrige Phase mit den Natriumsalzen der organischen Säuren abgetrennt und nach Entfärbung mittels Aktivkohle oder Adsorbeharzen wird diese Lösung konzentiert, und man erhält ca. 220 g eines Feuchthaltemittels mit ca. 50 % Trockensubstanz.

Ausführungsbeispiel 2:

In eine Glaskolonne mit 50 mm Durchmesser werden 1 l eines makroporösen starksauren Kationenaustauschers eingefüllt und, wie üblich, mit Schwefelsäure regeneriert, nachgewaschen und rückgespült. Auf die so vorbereitete Trennkolonne wird eine teilentzuckerte Melasse aus der Melassetrennung mittels Ionenausschlußchromatographie mit ca. 10 % Trockensubstanz aufgegeben und am Auslauf der Kolonne Fraktionen von je 1 l abgezogen. Die ersten 4 l vom pH-Wert 1,0 bis 2,5 werden für die Gewinnung der organischen Säuren verwendet und die nächsten 4 l von pH 2,5 bis 6,0 werden beim nächsten Trennzyklus erneut aufgegeben. Anschließend wird die Kolonne mit 1,5 l Wasser nachgewaschen und dann mittels 500 ml 1 molarer Natronlauge die Farbstoffe eluiert, erneut mit Wasser nachgewaschen und mit 1 l 1 molarer Schwefelsäure regeneriert. Bei der Regeneration werden gleichfalls 2 Fraktionen abgezogen. Die erste Fraktion enthält vorwiegend Kaliumsulfat und die zweite Fraktion vorwiegend freie Schwefelsäure. Die zweite Fraktion wird für die Vorregeneration beim nächsten Zyklus verwendet. Die erste Fraktion vom Kationenaustauscher mit den organischen Säuren wird konzentriert und mit der doppelten menge Butanol extrahiert. Die Butanolphase mit den freien organischen Säuren wird mit 1 molarer Natronlauge neutralisiert, Butanol abgetrennt und erneut in den Extraktionsprozeß zurückgeführt. Die wässrige Phase mit den Salzen der oranischen Säuren wird auf ca. 250 g konzentriert, und ein Feuchthaltemittel mit ca. 38 % Pyrrolidondarbonsäure und ca. 25 % Milchsäure in der Trockensubstanz gewonnen.

Ausführungsbeispiel 3:

1 Liter makroporöser schwachbasischer Anionenaustauscher wird in eine Glaskolonne mit 50 mm Durchmesser eingefüllt und, wie üblich, mit Ammoniak regeneriert, nachgewaschen und rückgespült. Über diesen Anionenaustauscher werden dann 4 l Auslauf vom Kationenaustauscher mit pH 1,0 bis 2,5 mit einer Geschwindigkeit von 5 Bettvolumen pro Stunde geleitet. Am Auslauf des Austauschers wird eine Zuckerlösung mit 4 % Trockensubstanz und einem pH-Wert von 7,0 bis 7,5 abgezogen. Bei Erreichung eines pH-Wertes von 6,0 im Auslauf wird der Austauscher mit 1,5 l Wasser gewaschen und die organischen Säuren mit 0,5 l 1 molarer Schwefelsäure eluiert und anschließend mit 1,5 l Wasser nachgewaschen. Nach dem Waschen wird der Anionenaustauscher mit 1 l 2 molarer Ammoniaklösung regneriert, mit Wasser nachgewaschen und rückgespült. Das saure Eluat vom Anionenaustauscher mit den organischen Säuren und etwas freier Schwefelsäure wird mit Kalziumcarbonat auf einen pH-Wert von 2,0 eingestellt, konzentriert und nach Neutralisation mit Natronlauge das auskristallisierte Kalziumsulfat abfiltriert. Man erhält ca. 300 g eines Feuchthaltemittels.

Dieses Feuchthaltemittel hat folgende Zusammensetzung:

```
Analysenwerte auf 100 % Trockensubstanz

Natrium-Pyrrolidoncarboxylat      ca. 35 %

Natrium Lactat                    ca. 35 %

Natrium Citrat       ⎫
Natrium Malat        ⎬            ca. 25 %
Natrium Acetat       ⎭
Natrium Formiat

Mineralsalze und                  ca.  5 %
andere Substanzen
```

**Patentansprüche**

1. Verfahren zur Herstellung eines Feuchthaltemittels, **dadurch gekennzeichnet**, daß zuckerfreie oder teilentzuckerte Restmelasse aus Zuckerrübenmelasse einer Behandlung unterworfen wird, bei der enthaltene Kaliumsalze von organischen Säuren, insbesondere von L-2-Pyrrolidon-5-Carbonsäure und von Milchsäure, in diese freien Säuren überführt, die Nebenprodukte von den freien Säuren getrennt, diese neutralisiert, entfärbt und zu einem Feuchthaltemittel konzentriert werden.

2. Verfahren nach Anspruch 1, **dadurch gekennzeichnet,** daß die Restmelasse auf einen pH-Wert von mindestens 3,0, insbesondere von 1,5 bis 2,0, angesäuert und mit einem organischen Lösungsmittel die organischen Säuren, insbesondere L-2-Pyrrolidon-5-Carbonsäure und Milchsäure, extrahiert, neutralisiert und zu einem Feuchthaltemittel konzentriert werden.

3. Verfahren nach Anspruch 1, **dadurch gekennzeichnet,** daß die Restmelasse über einen Kationenaustauscher geführt und die Kaliumionen an diesen gebunden und anschließend die entstehenden freien organischen Säuren extrahiert werden.

4. Verfahren nach Anspruch 1, **dadurch gekennzeichnet,** daß die Restmelasse über einen Kationenaustauscher geführt und die Kaliumionen an diesen gebunden und anschließend das verbleibende Gemisch über einen Anionenaustauscher geführt und die entstandenen freien organischen Säuren an diesen gebunden werden, daß das jetzt verbleibende Gemisch entfernt wird und anschließend die organischen Säuren, insbesondere L-2-Pyrrolidon-5-Carbonsäure und Milchsäure, vom vom Anionenaustauscher eluiert, neutralisiert und zu einem Feuchthaltemittel konzentiert werden.

5. Verfahren nach einem der vorstehenden Ansprüche, **dadurch gekennzeichnet,** daß zur Extraktion als organisches Lösungsmittel Butanol oder Isobutanol eingesetzt wird.

6. Verfahren nach einem der vorstehenden Ansprüche, **dadurch gekennzeichnet,** daß zum Ansäuern der Restmelassen und/oder zum Eluieren vom Anionenaustauscher Schwefelsäure verwendet wird.

7. Verfahren nach Anspruch 6, **dadurch gekennzeichnet,** daß die Restmelasse erhitzt und gemeinsam mit dem auskristallisierten Kaliumsulfat vor der Extraktion mit dem organischen Lösungsmittel die Farbstoffzersetzungsprodukte abgetrennt werden.

8. Verfahren nach einem der Ansprüche 4 bis 7, **dadurch gekennzeichnet,** daß die Ansäuerung der Restmelassen über einen starksauren Kationenaustauscher erfolgt, die adsorbierten Farbstoffe mittels Alkalihydroxiden vor der Regeneration eluiert und bei der Regeneration mit Schwefelsäure eine Kaliumsulfatlösung gewonnen wird.

9. Verfahren nach einem der Ansprüche 4 bis 8, **dadurch gekennzeichnet**, daß die Abläufe aus dem Kationenaustauscher über einen schwachbasischen Anionenaustauscher geleitet und als erste Fraktion eine Zuckerlösung bis pH 6,0 abgetrennt und anschließend die organischen Säuren mittels verdünnter Mineralsäure oder Alkalihydroxiden eluiert werden.

## Claims

1. Process for manufacturing a humectant, **characterised in that** sugar-free or partially desugarized residual molasses from sugar beet molasses is subjected to a treatment in which included potasssium salts of organic acids, in particular of L-2-pyrrolidone-5-carboxylic acid and of lactic acid, are converted into said free acids, the by-products are separated from the free acids, which are neutralized, decolorized and concentrated to form a humectant.

2. Process according to claim 1, **characterised in that** the residual molasses is acidified to a pH value of at least 3.0, in particular of 1.5 to 2.0, and with an organic solvent the organic acids, in particular L-2-pyrrolidone-5-carboxylic acid and lactic acid, are extracted, neutralized and concentrated to form a humectant.

3. Process according to claim 1, **characterised in that** the residual molasses is passed through a cation exchanger and the potassium ions are bonded to the latter and after this the free organic acids formed are extracted.

4. Process according to claim 1, **characterised in that** the residual molasses is passed through a cation exchanger and the potassium ions are bonded to the latter and after this the mixture remaining behind is passed through an anion exchanger and the free organic acids formed are bonded to the latter, that the mixture now remaining behind is removed and after this the organic acids, in particular L-2-pyrrolidone-5-carboxylic acid and lactic acid, are eluted from the anion exchanger, neutralized and concentrated to form a humectant.

5. Process according to any one of the preceding claims, **characterised in that** for the extraction butanol or iso-butanol is used as an organic solvent.

6. Process according to any one of the preceding claims, **characterised in that** for the acidification of the residual molasses and/or for the elution from the anion exchanger sulphuric acid is used.

7. Process according to claim 6, **characterised in that** the residual molasses is heated and, jointly with the crystallized out potassium sulphate prior to the extraction with the organic solvent, the pigment decomposition products are separated.

8. Process according to any one of claims 4 to 7, **characterised in that** the acidification of the residual molasses takes place by means of a strongly acid cation exchanger, the adsorbed pigments are eluted by means of alkali hydroxides prior to the regeneration and during the regeneration with sulphuric acid a potassium sulphate solution is obtained.

9. Process according to any one of claims 4 to 8, **characterised in that** the effluents from the cation exchanger are passed through a weakly basic anion exchanger and as a first fraction a sugar solution up to pH 6.0 is separated and after this the organic acids are eluted by means of dilute mineral acid or alkali hydroxides.

**Revendications**

1. Procédé pour la fabrication d'un agent hydratant, caractérisé en ce que l'on soumet à un traitement une mélasse finale exempte de sucre ou en partie exempte de sucre, tirée de la mélasse de betterave à sucre, traitement au cours duquel on réduit les sels de potassium des acides organiques contenus dans la mélasse, en particulier les sels de potassium de l'acide L-2-pyrrolidone-5-carboxylique et de l'acide lactique en leurs acides respectifs, on sépare les acides non liés de leurs sous-produits, on les neutralise, on les décolore et on les concentre pour obtenir un agent hydratant.

2. Procédé d'après la revendication 1, caractérisé en ce que l'on acidifie la mélasse finale pour atteindre une valeur de pH d'au minium 3,0, valeur particulièrement comprise dans un intervalle allant de 1,5 à 2,0 et en ce que l'on extrait à l'aide d'un solvant organique les acides organiques, en particulier l'acide L-2-pyrrolidone-5-carboxylique et l'acide lactique, en ce qu'on les neutralise et en ce qu'on les concentre pour obtenir un agent hydratant.

3. Procédé d'après la revendication 1, caractérisé en ce que l'on amène la mélasse finale au contact d'un échangeur de cations, en ce que les ions de potassium se fixent sur celui-ci et que l'on extrait immédiatement après les acides organiques libérés.

4. Procédé d'après la revendication 1, caractérisé en ce que l'on amène la mélasse finale au contact d'un échangeur de cations et que les ions de potassium se fixent à celui-ci et en ce que l'on amène immédiatement après le mélange restant au contact d'un échangeur d'anions et que les acides organiques libérés se fixent sur celui-ci, en ce que l'on élimine le mélange restant encore et qu'on élue immédiatement après les acides organiques, en particulier l'acide L-2-pyrrolidone-5-carboxylique et l'acide lactique de l'échangeur d'anions, qu'on les neutralise et qu'on les concentre de façon à obtenir un agent hydratant.

5. Procédé d'après l'une des revendications précédentes, caractérisé en ce que l'on emploie du butanol ou de l'isobutanol comme solvant organique pour l'extraction.

6. Procédé d'après l'une des revendications précédentes, caractérisé en ce que l'on emploie de l'acide sulfurique pour acidifier les mélasses finales et/ou pour les éluer de l'échangeur d'anions.

7. Procédé d'après la revendication 6, caractérisé en ce que l'on chauffe la mélasse finale et que l'on sépare les produits de décomposition des matières colorantes en même temps que le sulfate de potassium cristallisé, avant l'extraction par le solvant organique.

8. Procédé d'après l'une des revendications 4 à 7, caractérisé en ce que l'on effectue l'acidification des mélasses finales au contact d'un échangeur de cations fortement acide, qu'avant la régénération, on élue au moyen d'hydroxydes alcalins les matières colorantes adsorbées et que, lors de la régénération par l'acide sulfurique, on récupère une solution de sulfate de potassium.

9. Procédé d'après l'une des revendications 4 à 8, caractérisé en ce que l'on amène les émissions en provenance de l'échangeur de cations au contact d'un échangeur d'anions faiblement basique et que l'on sépare en tant que première fraction une solution sucrée jusqu'à ce que l'on ait atteint une valeur de pH de 6,0 et que l'on élue immédiatement après les acides organiques en utilisant un acide minéral dilué ou un hydroxyde alcalin dilué.